(19) Europäisches Patentamt / European Patent Office / Office européen des brevets

(11) **EP 3 381 547 A1**

(12) **EUROPEAN PATENT APPLICATION**

(43) Date of publication:
**03.10.2018 Bulletin 2018/40**

(51) Int Cl.:
*B01J 19/12* (2006.01)  *G01N 21/62* (2006.01)
*G01N 21/63* (2006.01)  *G01N 21/76* (2006.01)
*G01N 21/77* (2006.01)  *G01N 21/84* (2006.01)

(21) Application number: **18165209.0**

(22) Date of filing: **29.03.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2017 JP 2017068553
16.03.2018 JP 2018048873**

(71) Applicant: **Canon Medical Systems Corporation
Tochigi 324-8550 (JP)**

(72) Inventors:
• **YAMADA, Keiju
Tokyo, 105-8001 (JP)**
• **ITOU, Takayoshi
Tokyo, 105-8001 (JP)**
• **KANAYAMA, Shoichi
Tochigi (JP)**
• **IKEDA, Naru
Tochigi (JP)**
• **HARAGASHIRA, Motoji
Tochigi (JP)**

(74) Representative: **Moreland, David
Marks & Clerk LLP
Aurora
120 Bothwell Street
Glasgow G2 7JS (GB)**

(54) **SPECIMEN TEST APPARATUS**

(57) According to one embodiment, a specimen test apparatus includes a resonator (2), a detector (3), a radiator (22), and a dielectric (24). The resonator (2) is configured to house a test container (21) filled with a test solution (211). The detector (3) detects a detection target substance contained in the test solution (211) in the test container (21). The radiator (22) is arranged in the resonator (2) and emits electromagnetic waves, which resonate in a specific resonance direction in the resonator (2), into the resonator (2). The dielectric (24) is arranged in the resonator (2) at a position near the test container (21) when the test container (21) is placed in the resonator (2).

FIG. 2

EP 3 381 547 A1

**Description**

FIELD

Embodiments described herein relate generally to a specimen test apparatus.

BACKGROUND

A specimen test apparatus is configured to detect and quantify a detection target substance contained in a test solution obtained by mixing a specimen collected from a living body and a test reagent, and is used in the medical field to test various illnesses and infectious diseases., In order to make an accurate determination and apply an appropriate treatment at an early stage based on the result of the test, it is required to detect and quantify the detection target substance with high sensitivity and in a short time. That is, it is important for the performance of the specimen test apparatus to improve the sensitivity and quantitativeness as well as to shorten the test time.

The detection target substance reacts with a test reagent corresponding thereto and thereby can be detected. The detection target substance is detected by a detection method (various optical detection methods or electromagnetic detection methods) corresponding thereto and quantified. By promoting the reaction between the detection target substance and the test reagent, it is possible to improve the sensitivity and quantitativeness as well as to shorten the test time. The reaction between the detection target substance and the test reagent may be promoted by, for example, controlling the temperature of the test solution (mixture of the specimen and the test reagent).

Besides, in the case of testing a small amount of a specimen (mucous membrane, saliva, blood, etc.) collected from the human body, the volume of the test solution is likely to be a few mL to 1 mL or less. In order to promote the reaction between the detection target substance and the test reagent in such a small amount of the test solution, the specimen test apparatus needs a temperature control mechanism for rapidly heating and accurately controlling a small amount of the test solution.

Examples of the temperature control mechanism include heating devices such as resistive electric heaters and Peltier devices. When used, the heating device is provided outside a test container filled with the test solution. In this case, external heating is performed by heating the test solution through the test container from the outside. Accordingly, the heating efficiency is low, and it is difficult to control the temperature for rapid heating, reduction of overshoot, and the like. There has also been known a method of using a heating device and a thermostatic bath in combination. Similarly, in this method, external heating is performed and it is difficult to rapidly heat and precisely control the test solution. In addition, it is difficult to heat uniformly in a short time because of a temperature difference between the test container and the test solution. Further, since the heating device is installed outside the test solution, the size of the device is inevitably increased.

High-frequency heaters and microwave chemical reaction promotion devices are also known. These devices use heat generation when electromagnetic waves in a specific wavelength range such as, for example, microwaves are absorbed by a liquid. For example, the high-frequency heater discharges microwaves generated by a high-power high-frequency source such as a magnetron to a heating chamber such that the microwaves are directly absorbed by an object to be heated in the heating chamber, thereby internally heating the object. Since the high-frequency heater causes the electric energy to be directly absorbed by the object to be heated, higher energy transmission efficiency can be achieved as compared to the external heating. Moreover, since temperature rise/fall can be switched appropriately by on/off switching control of microwaves, the accuracy of temperature controllability is higher as compared to the external heating.

However, in the case of a small amount of test solution (object to be heated), the absorption efficiency of microwaves decreases. In order to rapidly heat a small amount of test solution by microwaves, in other words, to cause the test solution to absorb microwaves with high efficiency, the internal dimension of the heating chamber are set to allow microwave resonance to make the heating chamber a resonator. At this time, by placing the test solution at a position where the electric field intensity of the resonating microwaves is high, the absorption efficiency of the microwaves can be increased. In this case, the internal dimensions of the heating chamber (resonator) have to be not less than a half of the wavelength of the microwaves. Assuming that the frequency of the microwaves is 2.4 GHz, the wavelength is 12 cm, and the internal dimension of the resonator is required to be 6 cm or more. As described above, there is a concern that a large space is required to heat a small amount of the test solution, resulting in an increase in the size of the apparatus.

Further, the specimen test apparatus needs to be provided with a control arithmetic circuit, optical devices for detecting the detection target substance, and the like. Since the circuit and devices have to be arranged at positions not affected by the microwaves, the size of the apparatus further increases.

Thus, if the conventional high-frequency heater is simply applied to the specimen test apparatus, the size of the apparatus must be large. The compact specimen test apparatus for a small amount of test solution needs a small heating chamber and high heating efficiency of microwaves.

BRIEF DESCRIPTION OF THE DRAWINGS

Fig. 1 is a block diagram illustrating the overall configuration of a specimen test apparatus according to an embodiment;
Fig. 2 is a schematic diagram illustrating the configuration of a resonator according to the embodiment;
Fig. 3A is a schematic diagram illustrating the configuration of an open/close part according to the embodiment;
Fig. 3B is a schematic diagram illustrating the configuration of the open/close part according to the embodiment;
Fig. 4 is a schematic diagram illustrating a flow of electric flux according to the embodiment;
Fig. 5A is a schematic diagram illustrating the arrangement of a plurality of dielectrics according to the embodiment;
Fig. 5B is a schematic diagram illustrating the arrangement of a plurality of the dielectrics according to the embodiment;
Fig. 6 is a schematic diagram illustrating the configuration of a detector according to the embodiment;
Fig. 7A is a schematic diagram illustrating the configuration of a resonator according to a first modification;
Fig. 7B is a schematic diagram illustrating the configuration of the resonator according to the first modification;
Fig. 8 is a schematic diagram illustrating the configuration of a magnetic field applying unit according to a second modification;
Fig. 9A is a schematic diagram illustrating the configuration of a dielectric and a test container according to a third modification; and
Fig. 9B is a schematic diagram illustrating the configuration of the dielectric and the test container according to the third modification.

DETAILED DESCRIPTION

In general, according to one embodiment, a specimen test apparatus includes a resonator, a detector, a radiator, and a dielectric. The resonator is configured to house a test container filled with a test solution. The detector detects a detection target substance contained in the test solution in the test container. The radiator is arranged in the resonator and emits electromagnetic waves, which resonate in a specific resonance direction in the resonator, into the resonator. The dielectric is arranged in the resonator at a position near the test container when the test container is placed in the resonator.

Illustrative embodiments are described in detail with reference to the drawings.

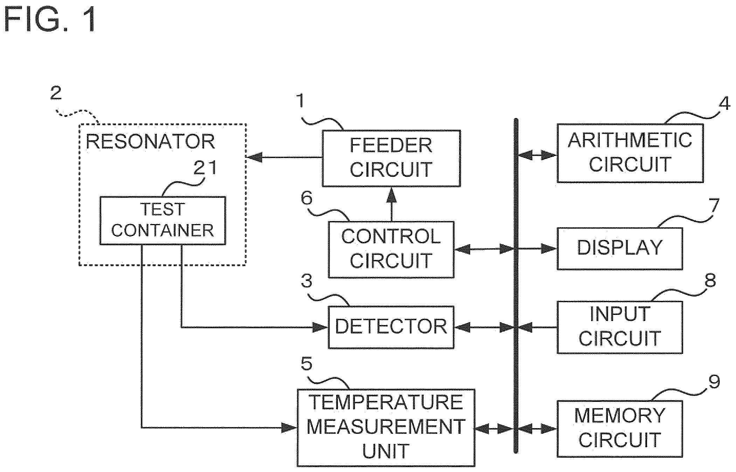

Fig. 1 is a functional block diagram illustrating the overall configuration of a specimen test apparatus according to an embodiment. The specimen test apparatus of the embodiment includes a feeder circuit 1, a resonator 2, a detector 3, an arithmetic circuit 4, a temperature measurement unit 5, a control circuit 6, a display 7, an input circuit 8, and a memory circuit 9. The detector 3 is an example of the detector in the claims.

The feeder circuit 1 includes a high frequency oscillator and a high frequency amplifier. The feeder circuit 1 is a circuit capable of supplying high-frequency waves of a predetermined frequency with a predetermined power. Examples of the predetermined frequency include 13.56 MHz band, 27.3 MHz band, 40.7 MHz band, 2.45 GHz band, 5.8 GHz band, 24.125 GHz band, and the like in the industry science medical (ISM) bands. The predetermined output power is determined as appropriate correspondingly to a test solution to be heated.

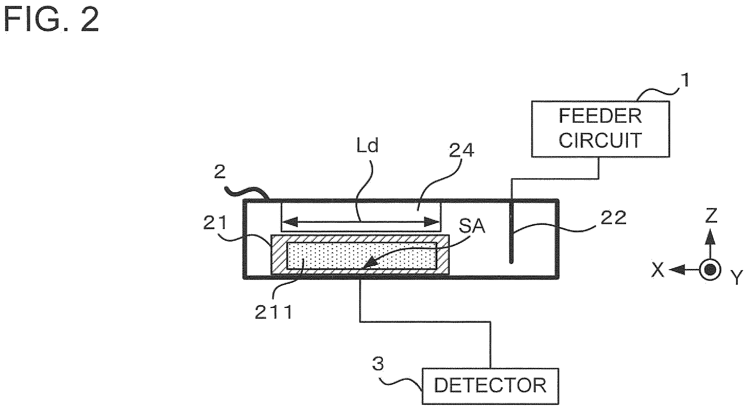

Fig. 2 is a schematic diagram illustrating the concept of the configuration of the resonator 2 according to the embodiment. The resonator 2 is a box-shaped structure that houses a test container 21 filled with a test solution. For example, the resonator 2 is configured such that the inner surface thereof is electrically conducted. This configuration may be obtained by, for example, forming the resonator 2 with a metal plate, with a nonmetallic plate material the inner surface of which is coated with a metal material. In the length in the resonator 2, the length in the resonance direction of electromagnetic waves is determined to be shorter than a half of the wavelength of the electromagnetic waves emitted (details will be described later).

The test container 21 is a box-shaped container that can be filled with a test solution 211. For example, the test container 21 is made of a material that allows high-frequency waves to pass through. As an example of this material may be cited a nonmetallic and low dielectric constant material, specifically, an insulator. The relative permittivity of the material of the test container 21 is, for example, in the range of 2 to 5, and the dielectric loss tangent is desirably 0.1 or less. Further, in the wall of the test container 21, the wall in a position close to the detector 3 during a test may be formed thin. Thereby, the distance between the detector 3 and the test solution 211 can be reduced.

The test solution 211 is a mixture of a specimen containing a test substance and a test reagent corresponding to the test substance. Examples of the test substance include a biomolecule corresponding to the measurement item of the test. The test reagent contains a detection target substance that specifically binds to the test substance. For example, if the test substance is a nucleic acid such as deoxyribonucleic acid (DNA) or ribonucleic acid (RNA), DNA which binds complementarily to the nucleic acid or a material which binds to DNA and can be optically or electromagnetically detected is used as the detection target substance. When the test substance is protein, bacteria, or the like, an antibody which specifically binds to the test substance or a material which binds to the antibody and can be optically or electromagnetically detected is used as the detection target substance. The relative permittivity of the test solution 211 varies in the range of about 10 to about 80 depending on the temperature of the test solution 211 and the frequency of high-frequency waves to be applied. Since the relative permittivity of the test solution 211 is higher than that of the test container 21, the high frequency waves tend to converge to the test solution 211. Further, if a frequency and a temperature with a high dielectric loss tangent of the test solution 211 of 0.1 or more are selected, the test solution 211 more easily absorbs the high frequency waves and is efficiently heated.

The test container 21 may be formed in a flat shape. The inner bottom surface of the flat test container 21 is referred to as a flat surface. The flat surface is provided with a sensor area SA in at least a part thereof. The sensor area SA is an area in which the detection target substance having bound to the test substance is fixed. A method of physically adsorbing or chemically adsorbing the detection target substance in the sensor area SA is used depending on the detection target substance to fix the detection target substance. The test container 21 is housed in the resonator 2 such that the flat surface is substantially parallel to the resonance direction of the electromagnetic waves (details will be described later).

A radiation pin 22 is provided inside the resonator 2. The radiation pin 22 is an example of the radiator in the claims. The radiation pin 22 radiates electromagnetic waves resonating in a specific resonance direction of the resonator 2 into the resonator 2. The radiation pin 22 is electrically connected to the feeder circuit 1 provided outside the resonator 2 through a via hole provided in the wall of the resonator 2. Examples of connection wiring between the radiation pin 22 and the feeder circuit 1 include high-frequency lines such as a coplanar line, a microstrip line, a strip line, and the like.

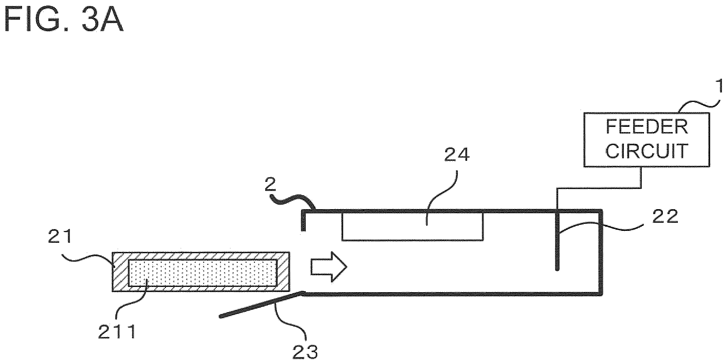

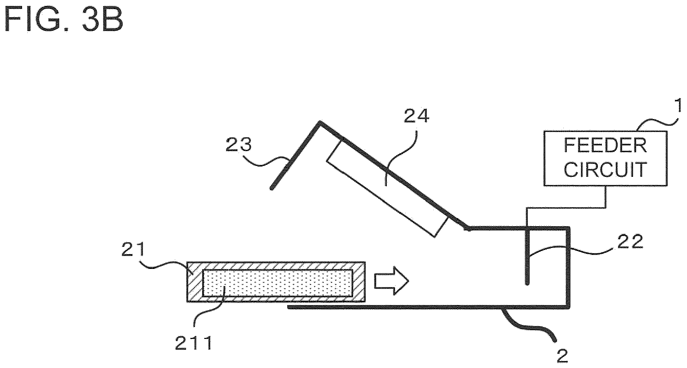

The resonator 2 has an open/close part 23 that opens and closes so that the test container 21 can pass therethrough when the test container 21 is installed in or removed from the resonator 2. Figs. 3A and 3B are schematic diagrams illustrating the configuration of the open/close part 23. For example, the open/close part 23 is formed of a hinge mechanism or the like to open and close a part of the wall of the resonator 2. With this, an operator such as a test technician can open the open/close part 23 to install the test container 21 inside the resonator 2, and close the open/close part 23. Such a configuration may be employed that when the open/close part 23 is open, the test container 21 is installed in a position away from a dielectric 24 (described later), while when the open/close part 23 is closed, the test container 21 moves to the vicinity of the dielectric 24 in conjunction with the closing operation. A general rotation/slide interlocking mechanism in which the opening/closing operation of the open/close part 23 is interlocked with the movement of the test container 21 may be applied to this configuration.

Although not illustrated, for another example, the open/close part 23 may be configured such that the resonator 2 is divided into a plurality of structures. In this case, the test container 21 may be installed at a predetermined position of any of the structures in a state where the resonator 2 is divided and housed inside the resonator 2 when the structures are combined.

The dielectric 24 is provided inside the resonator 2. The dielectric 24 is made of a material such as a ceramic plate. The dielectric 24 is arranged at a position near the test container 21 when the test container 21 is installed in the resonator 2. For example, a length Ld in the resonance direction of the electromagnetic waves in the length of the dielectric 24 is formed to be longer than the test solution 211. The dielectric 24 is directly or indirectly fixed to the inner wall of the resonator 2. Desirably, the dielectric 24 is brought into close contact with or close to the test container 21 so as to be as close as possible to the test solution 211 in a state where the test container 21 is placed in the resonator 2. With this, the electric field coupling between the dielectric 24 and the test solution 211 becomes stronger, and the capacitance between the dielectric 24 and the test solution 211 can be increased, thereby improving the heating efficiency of the test solution 211. In order to bring the dielectric 24 and the test container 21 into close contact with each other stably in a reproducible positional relationship, for example, a leaf spring (not illustrated) may be installed in a part of the resonator 2 so that the dielectric 24 can be directly or indirectly fixed to the leaf spring. Besides, the thickness of the dielectric 24 may be adjusted such that the electric field inside the resonator 2 is distributed so as to heat the test solution 211 with high efficiency. In that case, a spacer (not illustrated) is arranged between the dielectric 24 and the inner wall of the resonator 2 or between the dielectric 24 and the leaf spring structure on the resonator 2 side. With the use of an insulator having a dielectric constant lower than that of the dielectric 24 and a dielectric loss tangent of 0.1 or less for the spacer, it is possible to suppress the absorption of high-frequency energy by the spacer.

Described below is the relationship between heating of the test solution and electromagnetic waves. For the sake of explanation, first, a description is given of a case where the dielectric 24 is not placed in the resonator 2. An electromagnetic wave having a wavelength $\lambda$ is emitted from the radiation pin 22 to the inside of the resonator 2. When the relationship between the wavelength $\lambda$ and the width Wc is represented as follows:

$$(1/2)\,\lambda < Wc < \lambda \qquad \qquad \cdots (1)$$

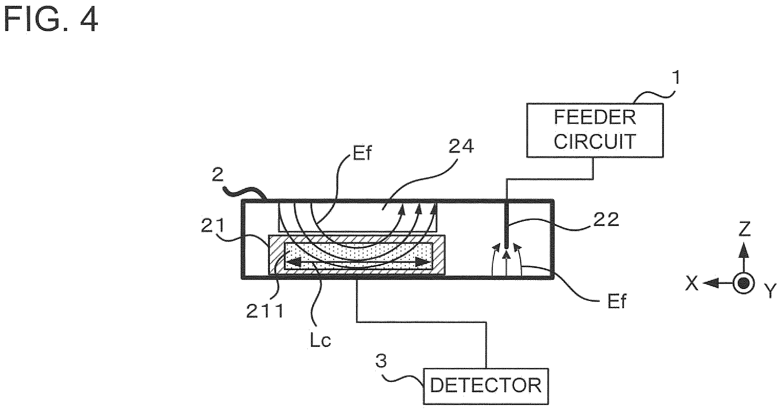

where Lc is the length in the resonator 2 in the X direction in Figs. 2 and 4 (the length in the resonance direction) and Wc is the width in the resonator 2 in the Y direction, the electromagnetic wave is in the state of single-mode resonance inside the resonator 2. In this state, the guide wavelength λg, which is the wavelength of the electromagnetic wave emitted into the resonator 2, is represented as follows:

$$\lambda g = \frac{\lambda}{\sqrt{1-(\lambda/2Wc)}} \qquad \cdots \qquad (2)$$

At this time, if the length Lc in the resonance direction in the resonator 2 is shorter than a half of the guide wavelength λg of the electromagnetic wave, that is, if the relationship between the length Lc and the guide wavelength λg is represented as follows:

$$Lc < (\lambda g / 2) \qquad \qquad \cdots (3)$$

the electromagnetic wave cannot enter the resonator 2. This results in a low efficiency of heating the test solution. In this case, it is required to increase the length Lc in the resonator 2 so that the electromagnetic wave can enter the resonator 2 to thereby improve the heating efficiency. Consequently, the size of the resonator 2 is increased.

Next, a description is given of a case where the dielectric 24 is placed in the resonator 2. When the following relationship is satisfied:

$$(Lc - Ld) + Ld\sqrt{\varepsilon_r} > \frac{\lambda_g}{2} \qquad \cdots \qquad (4)$$

where εr the relative permittivity of the dielectric 24 and Ld is the length of the dielectric 24 in the resonance direction, the wavelength of the electromagnetic wave is shortened by the dielectric 24. As a result, a standing wave is generated in the resonator 2, and the electromagnetic wave can enter the inside of the resonator 2. This indicates that the length Lc in the resonance direction in the resonator 2 can be shorter than a half of the guide wavelength λg of the electromagnetic wave. Fig. 4 is a schematic diagram illustrating a flow of electric flux Ef at this time. The electric field of the dielectric 24 is coupled with that of the resonator 2 and the test solution 211, and the electric field intensity between the dielectric 24 and the test solution 211 is increased. Thereby, a high-intensity electric field is applied to the test solution 211, enabling highly efficient heating of the test solution 211. Further, by making the length Ld of the dielectric 24 longer than the length Lc of the inside of the test container 21, i.e., the length of the test solution 211 in the same direction, the electric field is further concentrated on the test solution 211. Thus, the test solution 211 can be heated more efficiently. Further, the concentration of the electric field to the test solution 211 is enhanced by forming the flat surface in the test container 21 and the inner surface of the resonator 2 such that the flat surface and the resonance direction are substantially parallel to each other. The specific tolerance concerning this substantial parallelism may be determined as appropriate for each model.

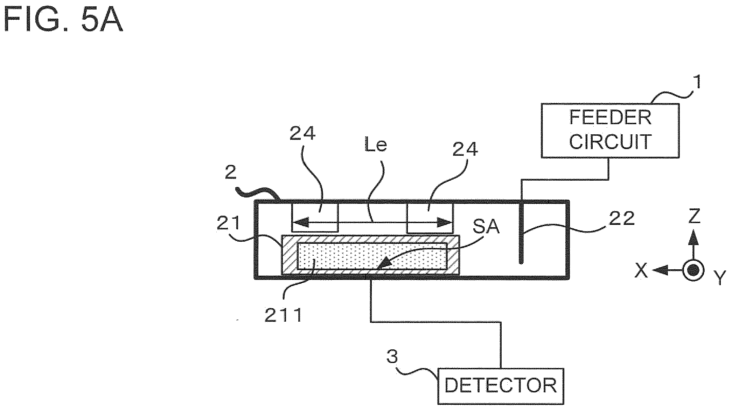

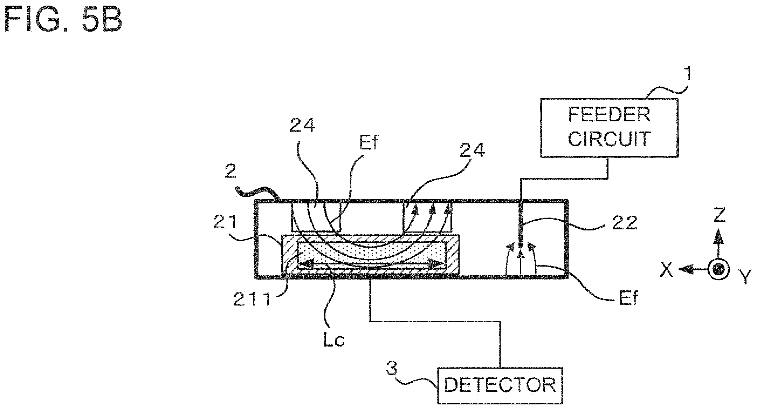

Further, a plurality of dielectrics (24) may be arranged at positions near the test container 21. Figs. 5A and 5B are schematic diagrams illustrating the arrangement of the dielectrics 24. In this case, in the arrangement of the dielectrics 24, the length Le between their outer edges in the resonance direction of electromagnetic waves may be determined to be the same as the length Ld described above. The dielectrics 24 allows resonating electromagnetic waves to reach the inside of the resonator 2. When the test container 21 is located at a position where the electromagnetic waves have an electric field of high intensity, the test solution 211 can be efficiently heated.

As described above, by arranging the dielectrics 24 near the test container 21, reduction of the resonator size and highly efficient heating of the test solution can be both achieved. In addition, since it is a highly efficient internal heating method using electromagnetic waves, there is a high interrelation between the timing of on/off control for the electromagnetic waves and the temperature of the test solution. Thus, the temperature can be controlled more precisely.

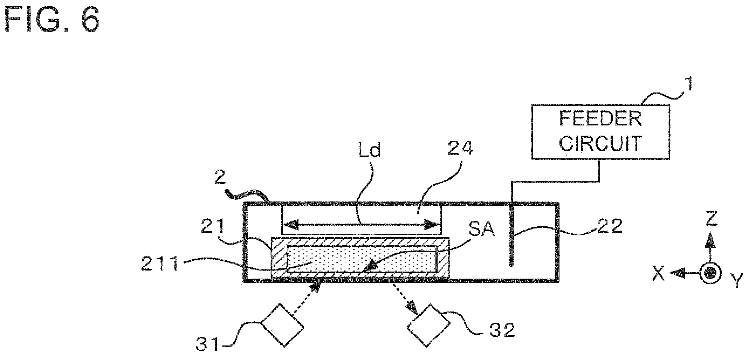

Fig. 6 is a schematic diagram illustrating the configuration of the detector 3. The detector 3 is configured to detect the detection target substance contained in the test solution in the test container 21. An optical method or an electromagnetic method is applied as appropriate to the detection of the detector 3 depending on the detection target substance. For example, the detector 3 includes a light source 31 and an optical detection unit 32 that optically detects the detection target substance. In this case, the detector 3 irradiates light to the sensor area side of the test container 21. The light propagates through the test solution 211 and the test container 21 on the sensor area side and reaches the optical detection unit 32. The optical detection unit 32 detects the light and generates detection light data indicating the intensity of the light detected. The optical detection unit 32 outputs the detection light data to the arithmetic circuit 4. The resonator 2 is provided with a window through which light can transmit or a hole through which light can pass in order to irradiate the test container 21 with the light such that the light can reach the optical detection unit 32. The position and size of the window or the hole are appropriately determined according to the positional relationship among the test container 21, the light source 31, and the optical detection unit 32. Further, the test container 21 may be provided with an optical waveguide that allows the light from the light source to reach the optical detection unit 32 in the wall on the sensor area SA side. The position and size of the optical waveguide are appropriately determined according to the positional relationship among the test container 21, the light source 31, and the optical detection unit 32.

The arithmetic circuit 4 includes a processor such as a central processing unit (CPU) and a microprocessor (MPU), and a memory such as a read only memory (ROM) and a random access memory (RAM). The arithmetic circuit 4 reads a predetermined control program from the memory circuit 9 and loads it into the memory. Upon receipt of the detection light data, the arithmetic circuit 4 performs predetermined quantitative analysis on the detection light data to calculate the amount and the concentration of the detection target substance contained in the test solution. The arithmetic circuit 4 controls each part of the specimen test apparatus according to the control program.

The temperature measurement unit 5 measures the temperature of the test solution 211. For example, in order to measure the temperature of the test solution 211 contained in the test container 21 during the application of electromagnetic waves, a non-contact type temperature measurement method using an optical sensor for temperature measurement is applied to the temperature measurement unit 5. The temperature measurement unit 5 sequentially outputs temperature data indicating the temperature measured to the control circuit 6.

The control circuit 6 is communicably connected to the feeder circuit 1. The control circuit 6 controls the output power, frequency, and the timing for applying and cutting off of high-frequency waves supplied from the feeder circuit 1 to the radiation pin 22. Various parameters related to this control may be preset or may be set by the operator through the input circuit 8.

As an example of the control of the control circuit 6, the control circuit 6 controls the feeder circuit 1 to emit electromagnetic waves from the radiation pin 22 and stop the electromagnetic waves after a lapse of a predetermined time. The predetermined time is an example of the various parameters related to the control.

For another example, the control circuit 6 controls the feeder circuit 1 to emit electromagnetic waves from the radiation pin 22 and reduce or stop the electromagnetic waves when the temperature measured by the temperature measurement unit 5 reaches a predetermined value. The predetermined temperature is an example of the parameters related to the control. The control circuit 6 sequentially receives the temperature data from the temperature measurement unit 5 and compares the temperature indicated by the temperature data with the predetermined temperature. Since the temperature indicated by the temperature data corresponds to the temperature of the test solution 211 being heated, the control circuit 6 recognizes that the temperature of the test solution 211 has reached the predetermined temperature by this temperature comparison. Then, the control circuit 6 can reduce or stop the electromagnetic waves so as to keep the temperature within a predetermined range. With the heating method using electromagnetic waves, the test solution 211 is directly heated from the inside, thereby achieving a quick rise in temperature. Therefore, these control examples enable fast heating with less overshoot.

For still another example, the control circuit 6 controls the feeder circuit 1 to repeat the emission and stop of electromagnetic waves from the radiation pin 22 at predetermined time intervals and stops the electromagnetic waves after a predetermined time has elapsed. The predetermined time intervals and the predetermined time are examples of the parameters related to the control. Depending on the type of the test solution 211, there are cases where it is desired to keep the temperature for a predetermined time after heating. According to this control example, after the test solution 211 is heated, the temperature can be kept for a predetermined time. At this time, the control circuit 6 may repeatedly emit and stop electromagnetic waves while performing feedback control with reference to the temperature data from the temperature measurement unit 5. Proportional-integral-differential (PID) control or the like may be applied to the feedback control. The control circuit 6 may also control the output intensity of electromagnetic waves. Thereby, the control circuit 6 can perform feedback control with higher accuracy.

The display 7 is a display device configured to display various data such as the detection result of the detection target substance and quantitative results. For example, various display devices such as a cathode ray tube (CRT) display, a liquid crystal display, an organic electroluminescence (EL) display, a light-emitting diode (LED) display, a plasma display, and the like can be appropriately used for the display 7.

The input circuit 8 is an operation device which is operated by an operator to provide various instructions to each part of the apparatus. The input circuit 8 may include various operation devices such as, for example, a button switch, a slide switch, a touch panel, a mouse, and a keyboard.

The memory circuit 9 is a storage device such as a hard disk drive (HDD), a solid state drive (SSD), an integrated circuit memory device, or the like. For example, the memory circuit 9 stores the data of quantitative analysis results. The memory circuit 9 also stores a control program and the like of the specimen test apparatus according to the embodiment.

<First Modification>

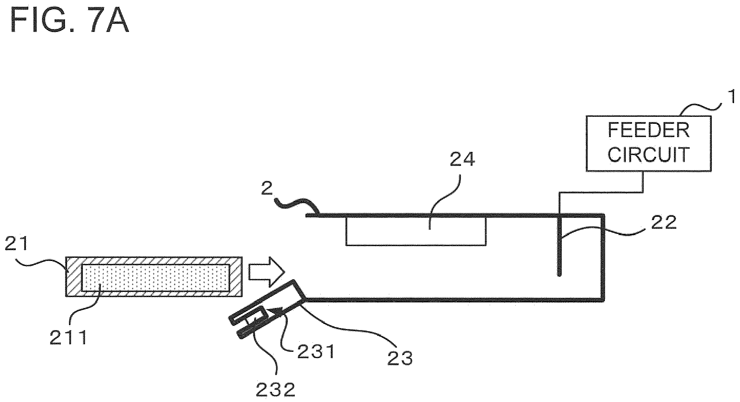

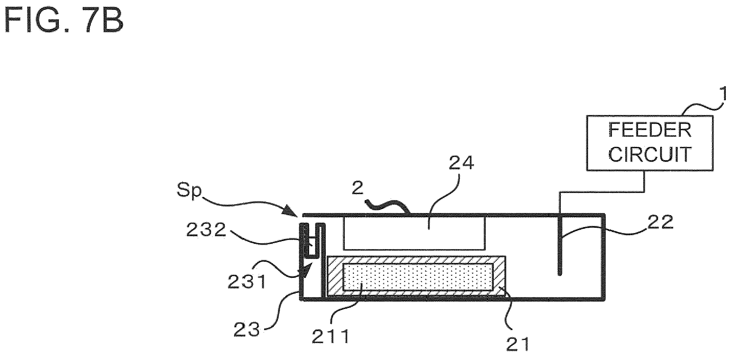

A specimen test apparatus according to a first modification is described. In the following, differences from the specimen test apparatus of the above embodiment are mainly described, and the same description may not be repeated. Figs. 7A and 7B are schematic diagrams illustrating the configuration of the resonator 2 of the specimen test apparatus according to the first modification. In the specimen test apparatus of the first modification, a groove 231 is formed in the open/close part 23.

The groove 231 is provided to prevent electromagnetic waves from leaking out of the open/close part 23. This is because a space Sp that communicates between the resonator 2 and the outside thereof may occur even in a state where the open/close part 23 is closed. The groove 231 is provided near this space. The shape and size of the groove 231 are determined such that the phase of electromagnetic waves traveling from the groove 231 side to the space Sp and the phase of electromagnetic waves traveling from the opposite side of the groove 231 to the space Sp are reversed to each other in the resonator 2. The shape and size of the groove 231 are appropriately determined according to electromagnetic waves assumed.

In addition, a dielectric member 232 may be provided in at least a part of the groove 231. The dielectric member 232 can shorten the wavelength of electromagnetic waves traveling from the groove 231 side to the space Sp. Thereby, the size of the groove 231 can be reduced, resulting in a reduction in the size of the resonator 2. The relative permittivity, shape, and size of the dielectric member 232 are appropriately determined according to electromagnetic waves assumed.

As described above, according to the first modification, it is possible to improve the speed of heating a small amount of test solution and the accuracy of the temperature control as well as preventing the leakage of electromagnetic waves.

<Second Modification>

A specimen test apparatus according to a second modification is described. In the following, differences from the specimen test apparatus of the above embodiment are mainly described, and the same description may not be repeated. The specimen test apparatus of the second modification includes a magnetic field applying unit.

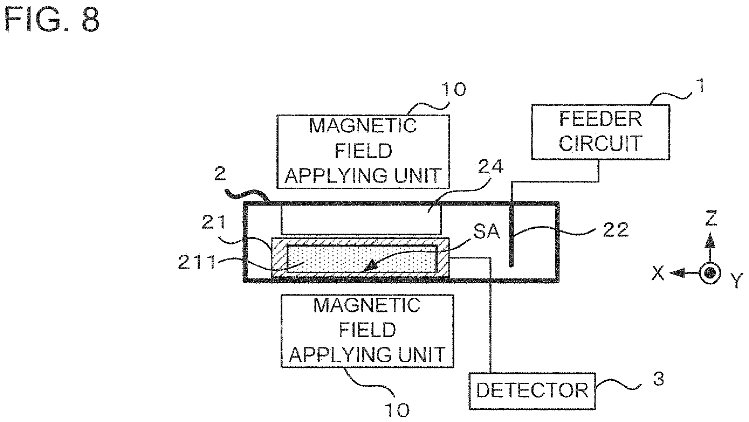

Fig. 8 is a schematic diagram illustrating the configuration of a magnetic field applying unit 10 according to the second modification. The magnetic field applying unit 10 applies a magnetic field from the outside of the resonator 2 into the test container 21. For example, the magnetic field applying unit 10 includes an electromagnet. The magnetic field applying unit 10 is provided on the outside of the upper portion or the lower portion of the resonator 2, or on the outside of both the upper and lower portions of the resonator 2. The resonator 2 is formed of a nonmagnetic metal.

For example, the magnetic field applying unit 10 is provided to a specimen test apparatus of a model for the test solution 211 that contains a magnetic substance. Depending on the detection target substance, there is a case where the magnetic substance specifically binds to the detection target substance or there is a case where the magnetic substance specifically binds to substances that may cause an error in the test in the test solution 211.

For example, in the case where the magnetic substance specifically binds to substances which may cause an error in the test, the magnetic field applying unit 10 is provided at least on the outside of the upper portion of the resonator 2. When the magnetic field applying unit 10 applies a magnetic field into the test container 21, an upward magnetic field is generated in the test container 21. Thereby, the magnetic substance having specifically bound to the substance which may cause an error in the test receives a force toward up in the test container 21, that is, toward the opposite side to the sensor area, and moves upward. In this manner, by removing a substance that may cause an error in the test from the sensor area to the opposite side, quantitative precision of the test can be improved.

Besides, for example, in the case where the magnetic substance specifically binds to the detection target substance, the magnetic field applying unit 10 is provided at least on the outside of the lower portion of the resonator 2. When the magnetic field applying unit 10 applies a magnetic field into the test container 21, a downward magnetic field is generated in the test container 21. As a result, the magnetic substance having specifically bound to the detection target substance receives a force toward down in the test container 21, that is, toward the sensor area, and moves downward. In this manner, by rapidly and forcibly collecting the detection target substance having bound to the magnetic substance in the sensor area, the test time can be shortened.

Further, when the magnetic field applying unit 10 is provided on both the upper and lower sides, the lower magnetic field applying unit 10 is turned on to rapidly collect the magnetic substance having bound to the detection target substance in the sensor area. Then, the lower magnetic field applying unit 10 is turned off, and the upper magnetic field applying unit 10 is turned on to remove the magnetic substance having bound to a substance which may cause an error in the test in the test solution 211. Thus, it is possible to detect only magnetic particles having bound to the detection target substance. This enables a shorter test time as well as a more sensitive test.

<Third Modification>

A specimen test apparatus according to a third modification is described. In the following, differences from the specimen test apparatus of the above embodiment are mainly described, and the same description may not be repeated. In the specimen test apparatus of the third modification, the dielectric 24 is provided in contact with the test container 21.

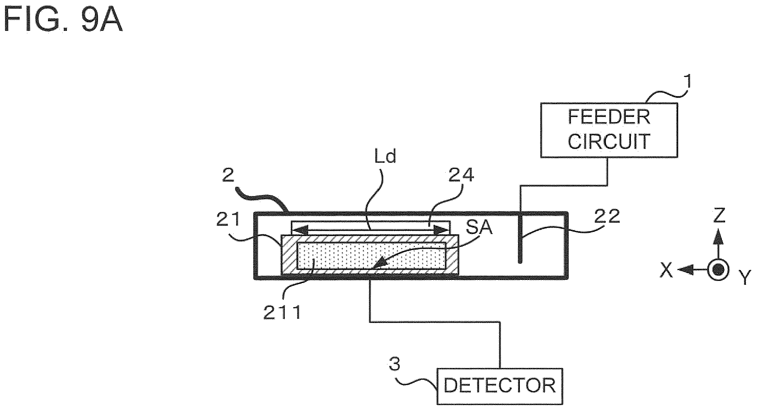

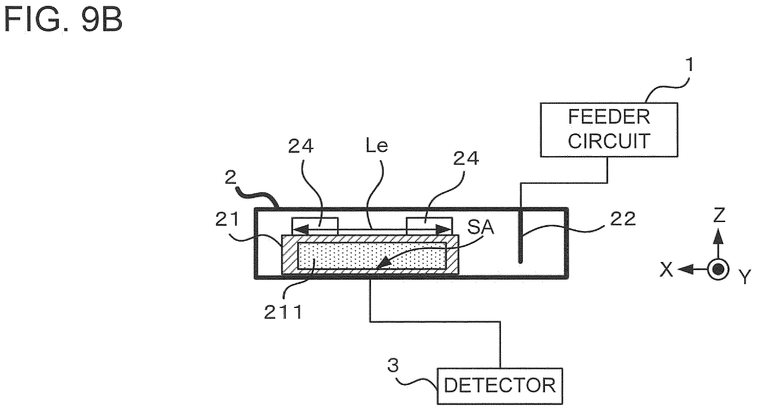

Figs. 9A and 9B are schematic diagrams illustrating the configuration of the dielectric 24 and the test container 21 according to the third modification. Fig. 9A illustrates an example in which there is provided one dielectric (24). Fig. 9B illustrates an example in which there are provided a plurality of dielectrics. The dielectric 24 is provided in contact with the outer edge of the test container 21 on the side opposite to the sensor area SA. For example, the dielectric 24 is affixed to the test container by an adhesive that does not interfere with the transmission of microwaves. The adhesive may be selected as appropriate.
In addition, the dielectric 24 is formed in a flat shape, and is affixed such that the flat surface of the dielectric 24 is parallel to the sensor area SA. With this, electric field concentration on the test solution 211 can be enhanced. Incidentally, the length Ld and the length Le may be determined in the same manner as in the above embodiment.
According to the third modification, the dielectric 24 is provided to the test container 21, not to the resonator 2. Therefore, the dielectric 24 can be formed in a size corresponding to the size of the test container 21 and affixed thereto. Thereby, specimen test can be performed while a plurality of types of test containers (21) are replaced one with another in one type of resonator (2).
The term "circuit" as used herein refers to, for example, CPU, a graphics processing unit (GPU), an application specific integrated circuit (ASIC), a programmable logic device including a simple programmable logic device (SPLD) and a complex programmable logic device (CPLD), a field programmable gate array (FPGA), or the like. The processor reads out, for example, a program stored in the memory circuit and execute it, thereby realizing the functions. The program may be directly incorporated in the circuit of the processor. In this case, the processor reads out the program incorporated in the circuit and execute it to realize the functions. Each processor of the embodiment need not necessarily be configured as a single circuit, but a plurality of independent circuits may be combined to form a single processor to realize the functions. Further, a plurality of constituent elements may be integrated into one processor to realize the functions.
According to at least one of the embodiment and modifications thereof described above, the specimen test apparatus is provided with a dielectric that is arranged in the resonator at a position near a test container when the test container is placed in the resonator. Thereby, it is possible to improve the speed of heating a small amount of test solution and the accuracy of the temperature control as well as preventing an increase in the size of the apparatus.
While certain embodiments have been described, these embodiments have been presented by way of example only, and are not intended to limit the scope of the inventions. Indeed, the novel embodiments described herein may be embodied in a variety of other forms; Further, various omissions, substitutions and changes in the form of the embodiments described herein may be made without departing from the spirit of the inventions. The accompanying claims and their equivalents are intended to cover such forms or modifications as would fall within the scope and spirit of the inventions.

**Claims**

1.   A specimen test apparatus, comprising:

a resonator configured to house a test container filled with a test solution;
a detector configured to detect a detection target substance contained in the test solution in the test container;
a radiator arranged in the resonator, configured to emit electromagnetic waves, which resonate in a specific resonance direction in the resonator, into the resonator; and
a dielectric arranged in the resonator at a position near the test container when the test container is placed in the resonator.

2.   The specimen test apparatus of claim 1, wherein a length in the resonance direction in the resonator is shorter than a half of wavelength of the electromagnetic waves emitted.

3. The specimen test apparatus of claim 1, wherein length of the dielectric direction is longer than length of inside of the test container in the resonance direction.

4. The specimen test apparatus of claim 1, wherein the dielectric includes a plurality of dielectrics arranged at positions near the test container.

5. The specimen test apparatus of claim 1, wherein
the resonator includes an open/close part configured to open and close to allow the test container to pass therethrough, and
a groove is formed in the open/close part.

6. The specimen test apparatus of claim 5, wherein a dielectric member is provided in at least a part of the groove.

7. The specimen test apparatus of claim 1, wherein
the detector includes an optical detector configured to optically detect the detection target substance, and
the resonator includes a window through which light to be detected by the optical detector transmits or a hole through which the light passes.

8. The specimen test apparatus of claim 1, wherein
the test container is formed in a flat shape,
a flat surface in the test container is provided with a sensor area to which a substance that specifically binds to the detection target substance or the detection target substance is fixed.

9. The specimen test apparatus of claim 8, wherein the dielectric is formed in a flat shape, and is provided in contact with the test container such that a flat surface of the dielectric is parallel to the sensor area.

10. The specimen test apparatus of claim 8, wherein the test container is housed in the resonator such that the flat surface is substantially parallel to the resonance direction.

11. The specimen test apparatus of claim 1, further comprising a magnetic field applying unit configured to apply a magnetic field into the test container,
wherein the resonator is formed of a nonmagnetic metal.

12. The specimen test apparatus of claim 1, further comprising:

a temperature measurement unit configured to measure temperature of the test solution, and
a controller configured to control time intervals of emission and stop of the electromagnetic waves and output power.

13. The specimen test apparatus of claim 1, wherein
the radiator is further configured to emit the electromagnetic waves after the test container is housed in the resonator, and stop the electromagnetic waves after a lapse of a predetermined time, and
after that, the detector detects the detection target substance.

14. The specimen test apparatus of claim 12, wherein
the radiator is further configured to emit the electromagnetic waves after the test container is housed in the resonator, and reduce or stop the electromagnetic waves when or after the temperature measured by the temperature measurement unit reaches a predetermined value to keep the temperature within a predetermined range, and
after that, the detector detects the detection target substance.

15. The specimen test apparatus of claim 1, wherein
the radiator is further configured to repeat emission and stop of the electromagnetic waves at predetermined time intervals after the test container is housed in the resonator, and stop the electromagnetic waves after a lapse of a predetermined time, and
after that, the detector detects the detection target substance.

# FIG. 1

RESONATOR 2

TEST CONTAINER 21

FEEDER CIRCUIT 1

CONTROL CIRCUIT 6

DETECTOR 3

TEMPERATURE MEASUREMENT UNIT 5

ARITHMETIC CIRCUIT 4

DISPLAY 7

INPUT CIRCUIT 8

MEMORY CIRCUIT 9

EP 3 381 547 A1

FIG. 2

EP 3 381 547 A1

**FIG. 3A**

FEEDER CIRCUIT

FIG. 3B

EP 3 381 547 A1

FIG. 4

# FIG. 5A

FIG. 5B

EP 3 381 547 A1

FIG. 6

FIG. 7A

EP 3 381 547 A1

EP 3 381 547 A1

# FIG. 7B

FEEDER
CIRCUIT

1

24

2

22

Sp

232

231

23

211

21

FIG. 8

MAGNETIC FIELD APPLYING UNIT 10

MAGNETIC FIELD APPLYING UNIT 10

FEEDER CIRCUIT 1

DETECTOR 3

24

22

SA

21

211

2

Z

X

Y

FIG. 9A

FEEDER CIRCUIT

1

2

Ld

24

SA

21

22

211

3

DETECTOR

Z

X

Y

EP 3 381 547 A1

EP 3 381 547 A1

FIG. 9B

Europäisches
Patentamt

European
Patent Office

Office européen
des brevets

# EUROPEAN SEARCH REPORT

Application Number

EP 18 16 5209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/093055 A1 (NAT INST FOR MATERIALS SCIENCE [JP]; MIYAZAKI HIDEKI [JP]; KUROKAWA YO) 8 September 2006 (2006-09-08) * paragraphs [0079] - [0104]; figures * ----- | 1-15 | INV. B01J19/12 G01N21/62 G01N21/63 G01N21/76 G01N21/77 G01N21/84 |
| X | US 2010/009458 A1 (OHTSUKA HISASHI [JP]) 14 January 2010 (2010-01-14) * paragraphs [0093] - [0111]; figures * ----- | 1,3-5, 7-10, 13-15 | |
| X | US 2009/321662 A1 (OHTSUKA HISASHI [JP]) 31 December 2009 (2009-12-31) * paragraphs [0091] - [0110]; figures * ----- | 1,3-5, 7-10, 13-15 | |

TECHNICAL FIELDS
SEARCHED (IPC)

B01J
G01N

The present search report has been drawn up for all claims

| Place of search | Date of completion of the search | Examiner |
|---|---|---|
| Munich | 16 August 2018 | Cagnoli, Michele |

EPO FORM 1503 03.82 (P04C01)

## ANNEX TO THE EUROPEAN SEARCH REPORT
## ON EUROPEAN PATENT APPLICATION NO.

EP 18 16 5209

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2018

| Patent document cited in search report | | Publication date | Patent family member(s) | | Publication date |
|---|---|---|---|---|---|
| WO 2006093055 | A1 | 08-09-2006 | JP<br>JP<br>WO | 4117665 B2<br>WO2006093055 A1<br>2006093055 A1 | 16-07-2008<br>07-08-2008<br>08-09-2006 |
| US 2010009458 | A1 | 14-01-2010 | JP<br>JP<br>US | 5190945 B2<br>2010019767 A<br>2010009458 A1 | 24-04-2013<br>28-01-2010<br>14-01-2010 |
| US 2009321662 | A1 | 31-12-2009 | JP<br>JP<br>US | 5301894 B2<br>2010008247 A<br>2009321662 A1 | 25-09-2013<br>14-01-2010<br>31-12-2009 |

EPO FORM P0459

For more details about this annex : see Official Journal of the European Patent Office, No. 12/82